# EUROPEAN PATENT SPECIFICATION

(11) **EP 2 156 172 B1**
(45) Date of publication and mention of the grant of the patent: **20.02.2013**
(21) Application number: 08795962.3
(22) Date of filing: 20.06.2008
(51) Int. Cl.: G01N 27/02, G01R 27/08, G01N 33/86, B01L 3/00

(54) **Apparatus for measuring blood coagulation**
Vorrichtung zur Messung der Blutgerinnung
Appareil pour mesurer la coagulation du sang

(30) Priority: 20.06.2007 US 945290 P
(43) Date of publication of application: 24.02.2010
(73) Proprietor: MEC Dynamics Corporation, San Jose, CA 95134 (US)
(72) Inventor: MPOCK, Emmanuel, C., San Jose, CA 95134 (US); MANGAN, Wilma, San Jose, CA 95134 (US)
(74) Representative: Round, Edward Mark
(86) International application number: PCT/US2008/067750
(87) International publication number: WO 2008/157795

(56) References cited:
- EP-A2- 0 974 840
- WO-A1-01/07892
- US-A- 4 858 127
- US-A- 5 073 057
- US-A1- 2005 233 466
- US-B1- 6 200 532
- US-B1- 6 750 053

## Description

### FIELD OF INVENTION

The present invention relates to microfluidics systems for performing assays for determining the presence of one or more selected analytes in a sample, in particular, for measuring blood coagulation.

### BACKGROUND

Blood clotting is a complex process involving three interacting components: blood vessels, blood coagulation factors, and blood platelets. There are two well-recognized coagulation pathways: the extrinsic or thromboplastin-controlled and the intrinsic or prothrombin/fibrinogen-controlled coagulation pathway. Both the extrinsic and intrinsic pathways result in the production of thrombin, a proteolytic enzyme which catalyzes the conversion of fibrinogen to fibrin. The soluble plasma protein fibrinogen is converted to insoluble fibrin by the action of the enzyme thrombin, resulting in a test sample changing from a liquid to a coagulated form.

In clinical assays, blood clotting assays measure the time required for the formation of a fibrin clot. There are many types of coagulation assays. These include prothrombin time (PT), partial thromboplastin time (PTT), activated partial thromboplastin time (APTT), fibrinogen assay, thrombin clotting time (TCT), activated clotting time (ACT), and others. The two most common coagulation tests are PT and APTT assays. Both tests measure clotting time to evaluate a patient's baseline hemostatic state or to monitor the response to anticoagulant therapy as well as the overall function and status of the coagulant system.

The PT test is used to assess the extrinsic and common pathway clotting systems and for monitoring long term anticoagulant therapy. A common medication for long term anticoagulant therapy is sodium warfarin isopropanol clathrate, generally known by the brand name COUMADIN™, sold by Bristol Myers Squibb. Warfarin and its analogs induce anticoagulation by effectively blocking biosynthesis of Vitamin K dependent coagulation factors. Since the PT test measures clotting time, the effective amount of anticoagulant in the blood can be determined.

The second routinely used assay, APTT test, is widely used for monitoring Heparin therapy for screening deficiencies of clotting factors included in the intrinsic and common coagulation system. Heparin exerts its anticoagulation effect by binding to and forming a complex with a plasma cofactor called antithrombin III.

In addition to the above, the FDA has approved several Protein C assay for clinical use that can broadly be grouped into three categories: antigenic, chromogenic/amidolytic, and coagulometric. The antigenic assays include ELISA, EIA and RIA type tests. These assays do not determine if the protein is functional because the antibodies are not directed to epitopes associated with functional activity.

The chromogenic/amidolytic assays rely on the ability of the active site of the enzyme to cleave a small synthetic substrate to release an intensely colored product. There may exist a discrepancy between the activity of activated Protein C towards a synthetic substrate and towards a natural biological substrate. In addition, other functional characteristics of the enzyme (i.e. cofactor interaction) are not tested by the synthetic substrate.

Coagulometric assays require generation of a standard curve prior to sample analysis. The samples require an initial dilution, an incubation for activation of the Protein C, then initiation of the clotting cascade. The standard curve between clot time and Protein C activity is used to interpolate the Protein C activity of the unknown sample. Although differences in laboratory procedure exist, a standard curve is routinely repeated with each group of patient samples tested, when new lots of reagent are opened or when the control does not fall within its prescribed range.

There are many disadvantages in the use of these methods. For example, the reagents are thermally sensitive, and require refrigeration prior to use. Once the dried reagent is reconstituted, it must be used within a few hours or discarded. Moreover, the laboratory instruments are relatively large because of the complex technology used, expensive, and designed for use by trained personnel due to the complexity of the detection methods. In addition, large blood samples are also usually required. Thus, there is a need for assay systems for detecting and determining the time for blood coagulation that are accurate, convenient, and inexpensive.

### SUMMARY

The present invention provides a cartridge comprising two reaction chambers, a blood reservoir, a reference port, and a sample application port, wherein the two reaction chambers are in fluidic communication with the blood reservoir via a capillary channel, the blood reservoir is in fluidic communication with the reference port via a capillary channel, the reference port is in fluidic communication with the sample application port via a capillary channel, and wherein the reservoir comprises a diaphragm for moving the samples within the capillary channels. In one embodiment the present invention provides apparatus and methods for performing assays for determining the time required for a sample of blood to coagulate. The apparatus comprises reaction chambers coated with one or more clotting agent. A drop of blood or equivalent is placed at the sample application port, and contacted with the clotting agents in the reaction chambers. Optionally, the sample can be diluted. The contacting can be by any means, such as, for example, by tilting the cartridge along the x, y, and z axis. The blood sample can move back and forth through the reaction chambers until blood clots. The blood clotting process forms fibrin stands that prevent the flow of the blood sample. The clotting time is the total time from the sample entering the reaction chambers to the time at which the motion or flow of the sample ceases or the amplitude of the waves generated within the capillaries change from that of the initial non clotting state.

These and other aspects of the present invention will become evident upon reference to the following detailed description.

### BRIEF DESCRIPTION OF DRAWINGS

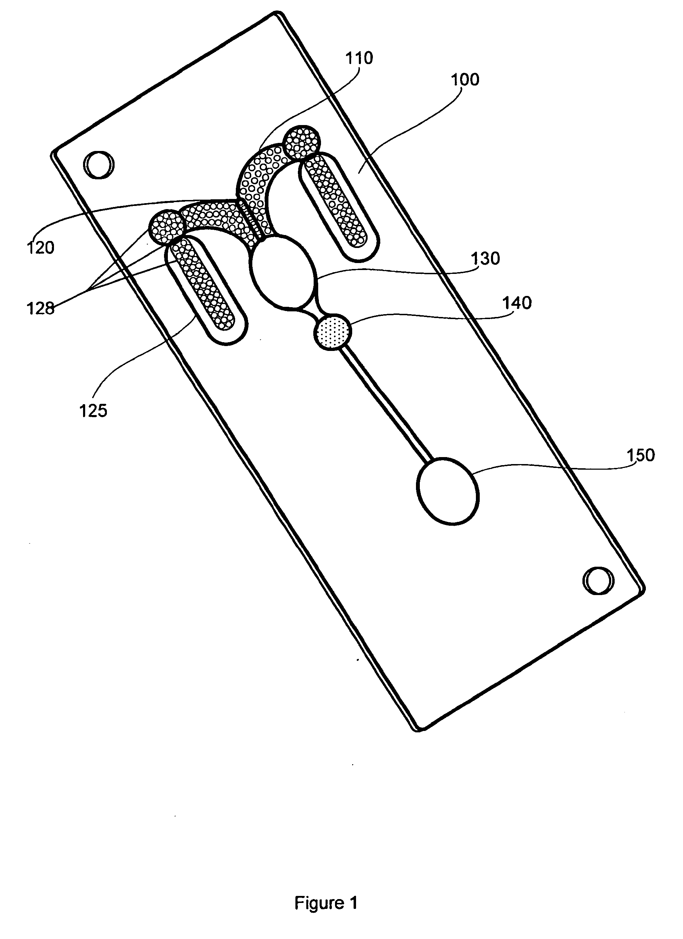

Figure 1 illustrates a perspective view of the disposable strip with a set of interconnected reaction chambers and a moveable member in one of the chambers.

### DETAILED DESCRIPTION

Unless otherwise stated, the following terms used in this application, including the specification and claims, have the definitions given below. It must be noted that, as used in the specification and the appended claims, the singular forms "a," "an" and "the" include plural referents unless the context clearly dictates otherwise.

As used herein, the term "subject" encompasses mammals and non-mammals. - Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. The term does not denote a particular age or gender.

As used herein, a "solid support" refers to a solid surface such as a plastic plate, magnetic bead, latex bead, microtiter plate well, glass plate, nylon, agarose, acrylamide, and the like.

Embodiments of the invention pertains to microfluidic apparatus for measuring blood coagulation time. A typical microfluidic appratus is illustrated in FIGURE 1. The apparatus comprises a cartridge **100** having reaction chambers 110 and 120 coated with one or more clotting agent such as tissue factor or other clotting agents. The reaction chambers **110** and **120** can be in fluidic communication via one or more capillary channels with the blood reservoir **130,** the non-clotting reference port **140,** and the sample application port **150.** A drop of blood or equivalent is placed at the sample application port **150.** Optionally, a diluent can be placed in the reservoir **150** whereby the mixing of the blood sample and the diluent can optionally provide a diluted blood sample. The blood sample can then be contacted with the clotting agents in the reaction chambers **110** and/or **120.** The contacting can be by any means, such as, for example, by mechanically moving a diaphragm of the reservoir **130** such that the sample in the capillary complex can be displaced at a given frequency and amplitude in synchronization with the diaphragm actuator, or by tilting the cartridge along the x, y, and z axis. The blood sample can move back and forth through the reaction chambers **110** and **120** until blood clots. The blood clotting process forms fibrin stands that prevent the flow of the blood sample and also changes the amplitude of the waveform resulting from the diaphragm actuation. The clotting time is the total time from the sample entering the reaction chambers **110** and **120** to the time at which the amplitude of the waveform changes and or motion or flow of the sample ceases.

The cartridge **100** can be rectangular, circular, oval, or any shape, and can be made from a suitable material that is selected on its properties, such as good thermal conductivity, clarity for optical transmission, mechanical properties for easy welding, surface properties that allow for uniform coating and stability of reagent, and neutrality to the liquid medium to prevent interference with the assay. For this purpose, suitable plastics include those with high free surface energies and low water sorption, including PETG, polyester (Mylar®), polycarbonate (Lexan®), polyvinyl chloride, polystyrene, SAN, acrylonitrile-butadiene-styrene (ABS), particularly ABS supplied by Borg Warner under the trade name Cycolac, among others. Alternatively and equivalently, a commercially-available molded cartridge can be used in the practice of the invention.

The cartridge **100** can have reaction chambers **110** and **120** coated with one or more clotting agent ,such as tissue factors. Each of the reaction chambers can be preferably exposed to the atmosphere. In one aspect of the invention, the reaction chambers **110** and **120** can have obstructions **128** distributed throughout. The obstructions **128** can be any shape, such as cylinders or pillars, with empty gaps in between that allow for the blood sample to flow back and forth until coagulation. In another aspect of the invention, the reaction chambers **110** and **120** do not have obstructions **128.**

The blood sample can be obtained from a patient by traditional means such as venipuncture or a finger prick. The sample can be applied via sample application port **150** onto the cartridge **100.** In one aspect of the invention, the sample of blood obtained from the patient can be used without additional manipulation in the methods and apparatus of the invention. Alternatively, the sample of blood obtained from the patient can be treated to remove, either completely or partially, the red blood cells. The red blood cells can be removed by any of the known methods, such as, for example, centrifugation, reacting the sample with a red blood cell agglutinant, or by employing a red blood cell filter. The use of plasma in conducting the methods can provide better accuracy and precision by, for example, allowing the imaging system to better monitor the physical changes taking place in the blood, such as the physical polymerization of the fibrinogen into fibrin.

The blood or the plasma can optionally be diluted with a diluent prior to coagulation. The diluent can simply be an aqueous solution or it can be a non-aqueous solution, and optionally can include various additives, such as, for example, salts, proteins, sugars, saccharides, metal ions, such as calcium, magnesium, lanthanides, and the like. Certain formulations of the diluent can include gelatin-containing compositions and emulsions. Typically, the diluent will be a buffer solution, such as citrate buffer. The diluent can be placed in the reservoir **130,** in the sample application port **150,** or in non-clotting well **140.**

The non-diluted sample or the diluted sample can then be moved into the reaction chambers **110** and **120** in order to perform the clotting assay. In one aspect of the invention, the sample can be moved using pressure. The reservoir **130,** for example, can be a curved element combined with a diaphragm or a flexible, compressible, deflatable, inflatable, or pressure moveable layer or element The diaphragm can be situated such that one surface of the diaphragm is exposed to the environment and a second surface of the diaphragm defines part of an internal cavity the reservoir **130,** and movement of the diaphragm causes changes in pressure within the internal cavity. The change in the pressure causes the movement of the blood sample into and out-of the reaction chambers **110** and **120,** and into and out-of the non-clotting reference port **140** and the sample application port **150.** Preferably, the diaphragm of the reservoir **130** can be moved mechanically such that the blood sample in the capillary complex is displaced at a given frequency and amplitude.

The clotting assays of an embodiment of the present invention include prothrombin time (PT), partial thromboplastin time (PTT), activated partial thromboplastin time (APTT), thrombin clotting time (TCT), fibrinogen, heparin management test (HMT), protamine response time (PRT), heparin response time (HRT), low molecular weight heparin (LMWH), low range heparin management test (LHMT), and ecarin clotting time (ECT), with the reagents for each of these tests as described in the art. The reagents for one or more the assays can be placed in one or both of the reaction chambers **110** and **120.** The reagents can be applied to all the surface of the reaction chambers or just the obstructions **128.** The reagents for the assays is placed in one of the reaction chambers **110** and **120,** one reaction chamber serving as an active control and reaction chamber **140** serving as the non-clotting control, since coagulation should not occur in that chamber. Alternatively, each of the reaction chambers **110** and **120** can have different reagents for the clotting assay. For example, the reaction chamber **110** can have the reagents for the PT assay, while the reaction chamber **120** can have the reagents for the HRT assay.

For example, if the clotting assay is PT, any source of thromboplastin can be used. For example, thromboplastin can be purchased from a commercial source or it can be extracted from human placenta, rabbit brain, bovine brain, ox brain, and human brain. Thromboplastin produced by recombinant DNA technology can also be used. Typically the source is rabbit brain. Rabbit brain powder is commercially available, and it can be isolated according to a standard technique by removing whole brains stripped of attached blood vessels from, for example, New Zealand White rabbits, homogenizing the rabbit whole brains in excess acetone in a Waring blender to produce a slurry, and drying the slurry under vacuum to produce rabbit brain powder which is stable when stored under vacuum at -20 °C. Powdered bovine brain, powdered ox brain powdered human brain, powdered thromboplastin produced by recombinant DNA technology, and powdered human placenta can be prepared in similar manner, or by well known art methods.

The powdered thromboplastin source such as rabbit brain powder or human placenta powder can then be extracted in an aqueous solution to prepare thromboplastin extract. The aqueous solution may be a warm saline solution that optionally contains a metal ion chelator. The metal ion chelator can be citrate, salts of citrate, ethylenediaminetetraacetic acid (EDTA), Ethylenebis(oxyethylenenitrilo)tetraacetic acid (EGTA), and salts thereof. The powdered brain material is extracted with the aqueous solution and thromboplastin separates into the supernatant. The supernatant can be centrifuged to isolate the thromboplastin, and the isolated thromboplastin can then be freeze dried.

The thromboplastin reagent can then be prepared by mixing the thromboplastin extract prepared by the above process with calcium ions. The thromboplastin reagent prepared by this process may but need not include the metal ion chelator in the final reagent. Any source of calcium ions may be used, such as, for example, calcium chloride, calcium tartrate, calcium gluconate, calcium citrate, or calcium lactate, and the like. The thromboplastin reagent thus prepared or obtained from a commercial source can be placed in the reaction chambers **110** and **120.**

In another example, the clotting assay can be the Protein C activator assay, and the methods and apparatus of the present invention are illustrated using the Protein C activator assay. This assay uses a reagent comprising an initiator of the intrinsic coagulation pathway or Factor X, a Protein C activator (such as thrombomodulin, Protac™, other catalytic or stoichiometric activators), calcium ions, one or more metal compounds that interact with calcium binding sites in the coagulation cascade, preferably one or more lanthanide compounds, and optionally bulkers and/or stabilizers.

The concentration of Protein C can be correlated to clot time, the rate of clot formation, the integrity of the fmal clot (i.e. clot strength), or any combination thereof. The assay can be calibrated to a standard control plasma to establish the relationship between the measured signal and the Protein C concentration. The standard control curve can be generated immediately prior to sample measurement, if desired, or can be a paper or electronically stored standard control curve.

The whole blood or plasma sample described above can be used as is or can be diluted in a Protein C depleted plasma, preferably 1 part sample to 5 parts depleted plasma, to reduce the concentration of interfering substances and replenish any coagulation factors that may be depleted due to a genetic or clinical condition. Also, if desired, polybrene or another heparin antagonist can be added to the diluted sample.

In the Protein C assay, the sample is located to the reaction chambers 110 and 120 containing the Protein C assay reagent. The present Protein C assay reagent comprises a Protein C activator, an initiator of the intrinsic coagulation pathway or of Factor X, calcium ions and optionally one or more lanthanide compounds.

In the Protein C assay, the reaction proceeds with an initial lag time during which it is believed that thrombomodulin is capturing thrombin and activating Protein C, and, the coagulation of the sample starts after about at least 200 seconds, preferably after about at least 250 seconds, more preferably after about at least 300 seconds. The initial lag time can vary from several seconds to around 5-7 minutes.

In the Protein C assay, the initiator of the intrinsic coagulation pathway or of Factor X can be any substance capable of triggering the coagulation cascade, such as ThromboScreen™ Kontact™, commercially available from Pacific Hemostasis of Huntersville, N.C. Other coagulation initiators of Factor X, prothrombin time (PT), and activated partial thromboplastin time (APTT) can also be used, including reagents reconstituted from purified components. The Protein C activator is preferably thrombomodulin prepared as describe above or obtained from a commercial source.

In another aspect, the apparatus can be used for the clotting and/or chromogenic testing for bacterial endotoxins. Bacterial endotoxins are mainly lipopolysacharide components derived from the cell membrane of Gram negative bacteria. The majority of these tests utilize Limulus Amebocyte Lysate (LAL) from the horseshoe crab *Limulus Polyphemus.* Exposure of LAL to bacterial endotoxins or glucans triggers a primitive clotting system resulting in the conversion of liquid LAL into a solidified gel clot similar to mammalian plasma clotting assays. This test, as well as any modifications utilizing alternate biological systems (i.e. *Tachypleus* amoebocyte lysate), purified components of these systems, recombinant proteins, alone or in combination with components of other clotting systems, structural proteins, protease inhibitors or activators are well adapted for use in the apparatus and method of the invention. Uses for this test system include but are not limited to quantitating endotoxin levels in parenteral pharmaceuticals, irrigation fluids, dialysis solutions, WFI, and medical devices as well as IVD products and process materials. The clotting and/or chromogenic testing for bacterial endotoxins using the apparatus also find utility in the monitoring of patients with endotoxaemia or sepsis.

In one aspect of the invention, the frequency and/or amplitude of the sample displacement in the reaction chambers **110** and **120** and the non-clotting reference port 140 can be measured as a function of time. The sample in the reference port **140** will not clot since the clotting agents are placed in the reaction chambers **110** and/or **120** only. The frequency and/or amplitude of the sample displacement in the reaction chambers **110** and **120** can be compared with the frequency and/or amplitude of the sample in the non-clotting reference port **140.** A change in the frequency and/or amplitude of the sample displacement in the reaction chambers **110** and **120** compared with the non-clotting reference port **140** is indicative of the transition of the sample from a liquid to a fibrous gel at the point of coagulation. Typically, the non-clotting reference port **140** can be left open to the atmosphere in order to buffer the force directed at a clotted reaction well, thereby allowing detection of the clot immediately it forms. Without the non clotting channel to absorb the additional force, weak trapped clots could be ruptured from the posts or cause delay in the detection thereof.

The coagulation time can be measured by detecting the degree of a physical change of a liquid sample which changes after the contact with a blood coagulation reagent. The physical change can be any change, such as, for example, turbidity (including absorbance), viscosity, permittivity and the like. Preferably, physical change is detected by measuring optically detecting the motion and or properties of the waves or by turbidity (or absorbance).

Typically, the membrane or the diaphragm on the reservoir **130** can be actuated vibrationally at a given frequency. The movement of the diaphragm moves the blood sample bodily across the optic path (along the length of the capillary) and the sinusoidal waves can be observed optically. When the gel forms (at the coagulation point) the fibrin mesh in the clot wraps around the posts and motion in the particular reaction well ceases. However motion in the reference port **140** continues and the optics match the wave motion in reference port **140** against the reaction chambers **110** and **120.** In another aspect, the reaction chamber **110** can serve as a control with a fixed clotting time while the reaction chamber **120** can serves as the test zone for the variable patient sample.

The reaction wells can be made deeper, to increase the size of the wave form, relative to the rest of the capillary system.

Other methods besides optics, can be used to detect the coagulated blood sample. For example, turbidity (or absorbance) of a liquid sample can be easily monitored optically, and the detection of the degree of turbidity change can be conducted easily using commercially available devises such as STAT IMUNO SYSTEM Quick Turbo II (manufactured by A & T Corp.), an automated analyzer Multiple Chemistry Unit 502X (manufactured by A & T Corp.), an automated analyzer Automatic Analyzer 7070 (manufactured by Hitachi Ltd.) and the like.

For example, the turbidity measurements can be performed using the automated analyzer Multiple Chemistry Unit 502X, where two wave lengths between 340 and 795 nm can be selected as the wave lengths for the measurements. The selected two wave lengths can be simultaneously measured through the intermittent measurements conducted in seconds.

## Claims

1. A cartridge comprising two reaction chambers (110, 120), a blood reservoir (130), a reference port (140), and a sample application port (150), wherein the two reaction chambers (110, 120) are in fluidic communication with the blood reservoir (130) via a capillary channel, the blood reservoir (130) is in fluidic communication with the reference port (140) via a capillary channel, the reference port (140) is in fluidic communication with the sample application port (150) via a capillary channel, and wherein the reservoir (130) comprises a diaphragm for moving the samples within the capillary channels.

2. The cartridge of claim 1, wherein the two reaction chambers (110, 120), the blood reservoir (130), the reference port (140) and the sample application port (150) are mounted on a substrate selected from the group consisting of plastic, glass, nylon, metal, and combinations thereof.

3. The cartridge of claim 1 or 2, wherein the reaction chambers (110, 120) comprise a clotting agent.

4. The cartridge of claim 3, wherein the clotting agent is for an assay selected from the group consisting of prothrombin time (PT), partial thromboplastin time (PTT), activated partial thromboplastin time (APTT), thrombin clotting time (TCT), fibrinogen, heparin management test (HMT), protamine response time (PRT), heparin response time (HRT), low molecular weight heparin (LMWH), low range heparin management test (LHMT), ecarin clotting time (ECT), and combinations thereof.

5. The cartridge of claim 4, wherein the assay is PT or APTT.

## Patentansprüche

1. Patrone, die aufweist: zwei Reaktionskammern (110, 120); einen Blutbehälter (130); eine Bezugsöffnung (140); und eine Probeneinsetzöffnung (150), wobei die zwei Reaktionskammern (110, 120) in Fluidverbindung mit dem Blutbehälter (130) mittels eines Kapillarkanals sind, der Blutbehälter (130) in Fluidverbindung mit der Bezugsöffnung (140) mittels eines Kapillarkanals ist, die Bezugsöffnung (140) in Fluidverbindung mit der Probeneinsetzöffnung (150) mittels eines Kapillarkanals ist, und wobei der Behälter (130) eine Membran für das Bewegen der Proben innerhalb der Kapillarkanäle aufweist.

2. Patrone nach Anspruch 1, bei der die zwei Reaktionskammern (110, 120), der Blutbehälter (130), die Bezugsöffnung (140) und die Probeneinsetzöffnung (150) auf einem Substrat montiert sind, das aus der Gruppe ausgewählt wird, die aus Kunststoff, Glas, Nylon, Metall und deren Kombinationen besteht.

3. Patrone nach Anspruch 1 oder 2, bei der die Reaktionskammern (110, 120) ein Gerinnungsmittel aufweisen.

4. Patrone nach Anspruch 3, wobei das Gerinnungsmittel für eine Analyse ist, die aus der Gruppe ausgewählt wird, die besteht aus: Prothrombinzeit (PT); partielle Thromboplastinzeit (PTT); aktivierter partieller Thromboplastinzeit (APTT); Thrombin-Gerinnungszeit (TCT); Fibrinogen; Heparin Management Test (HMT); Protaminansprechzeit (PRT); Heparinansprechzeit (HRT); Heparin mit niedriger relativer Molekülmasse (LMWH); Low-Range Heparin Management Test (LHMT); Ecarin-Gerinnungszeit (ECT); und deren Kombinationen.

5. Patrone nach Anspruch 4, bei der die Analyse PT oder APTT ist.

## Revendications

1. Cartouche, comprenant deux chambres de réaction (110, 120), un réservoir de sang (130), un orifice de référence (140) et un orifice d'application des échantillons (150), dans laquelle les deux chambres de réaction (110, 120) sont en communication de fluide avec le réservoir de sang (130) par l'intermédiaire d'un canal capillaire, le réservoir de sang (130) étant en communication de fluide avec l'orifice de référence (140) par l'intermédiaire d'un canal capillaire, l'orifice de référence (140) étant en communication de fluide avec l'orifice d'application des échantillons (150) par l'intermédiaire d'un canal capillaire et le réservoir (130) comprenant un diaphragme pour déplacer les échantillons dans les canaux capillaires.

2. Cartouche selon la revendication 1, dans laquelle les deux chambres de réaction (110, 120), le réservoir de sang (130), l'orifice de référence (140) et l'orifice d'application des échantillons (150) sont montés sur un substrat sélectionné dans le groupe constitué de plastique, de verre, de nylon, de métal et de combinaisons de ces matériaux.

3. Cartouche selon les revendications 1 ou 2, dans laquelle les chambres de réaction (110, 120) comprennent un agent de coagulation.

4. Cartouche selon la revendication 3, dans laquelle l'agent de coagulation est destiné à un essai sélectionné dans le groupe constitué du temps de prothrombine (PT), du temps de thromboplastine partiel (PTT), du temps de thromboplastine partiel activé (APTT), du temps de coagulation de la thrombine (TCT), de la fibrinogène, du test de gestion de l'héparine (HMT), du temps de réponse de la protamine (PRT), du temps de réponse de l'héparine (HRT), de l'héparine de faible poids moléculaire (LMWH), du test de gestion de l'héparine à faible dose (LHMT), du temps de coagulation de l'écarine (ECT) et des combinaisons correspondantes.

5. Cartouche selon la revendication 4, dans laquelle l'essai est un essai PT ou un essai APTT.
